# EUROPEAN PATENT SPECIFICATION

(11) **EP 4 164 669 B1**
(45) Date of publication and mention of the grant of the patent: **24.07.2024**
(21) Application number: 21734287.2
(22) Date of filing: 09.06.2021
(51) Int. Cl.: A61K 36/185, A61K 31/7048

(54) **PROCESS FOR THE PREPARATION OF AN EXTRACT OF EPILOBIUM SPP. WITH HIGH OENOTHEIN B CONTENT**
VERFAHREN ZUR HERSTELLUNG EINES EXTRAKTS VON EPILOBIUM SPP. MIT HOHEM OENOTHEIN-B-INHALT
PROCÉDÉ DE PRÉPARATION D'UN EXTRAIT D'EPILOBIUM SPP. À HAUTE TENEUR EN OENOTHÉINE B

(30) Priority: 11.06.2020 IT 202000014011
(43) Date of publication of application: 19.04.2023
(73) Proprietor: ISTITUTO FARMOCHIMICO FITOTERAPICO EPO S.R.L. ED IN FORMA ABBREVIATA: EPO S.R.L., 20141 Milano (MI) (IT)
(72) Inventor: NICOTRA, Giovanna, 20141 MILANO (MI) (IT); INSOLIA, Violetta, 20141 MILANO (MI) (IT); VICENTINI, Silvia, 20141 MILANO (MI) (IT); MARANO, Maria Grazia, 20141 MILANO (MI) (IT); BRUNO, Beatrice, 20141 MILANO (MI) (IT); DAGLIA, Maria, 80131 NAPOLI (NA) (IT)
(74) Representative: Bianchetti & Minoja with Trevisan & Cuonzo IPS SRL
(86) International application number: PCT/EP2021/065494
(87) International publication number: WO 2021/250110

(56) References cited:
- WO-A1-2009/054607
- JP-A- 2003 342 121
- N.N.: "Epilobium angustifolium L. dry extract standardized to contain 15% Oenothein B", 20 March 1995 (1995-03-20), pages 1 - 8, XP055779716, Retrieved from the Internet <URL:https://www.eposrl.com/wp-content/uploads/2019/03/Report-ENOTprost.pdf> [retrieved on 20210225]
- PIWOWARSKI JAKUB ET AL: "Abstract", vol. 83, no. 14/15, 28 April 2017 (2017-04-28), DE, pages 1159 - 1168, XP055779623, ISSN: 0032-0943, Retrieved from the Internet <URL:https://www.thieme-connect.de/products/ejournals/pdf/10.1055/s-0043-109372.pdf> DOI: 10.1055/s-0043-109372
- MAGDALENA STOLARCZYK ET AL: "Extracts from Epilobium sp. herbs induce apoptosis in human hormone-dependent prostate cancer cells by activating the mitochondrial pathway : Epilobium induce apoptosis in prostate", PHARMACEUTICAL AND CLINICAL RESEARCH, vol. 65, no. 7, 1 July 2013 (2013-07-01), GB, pages 1044 - 1054, XP055771481, ISSN: 0022-3573, DOI: 10.1111/jphp.12063

## Description

The present invention relates to a process for the preparation of an extract of *Epilobium* spp. with a high oenothein B content.

### State of the art

*Epilobium angustifolium* L. is a perennial herbaceous plant belonging to the *Onagraceae* family. It has simple, glabrous, upright stems and rhizomatous roots; the flowers are gathered in striking pyramid-shaped racemes of a pink to purple colour. The fruits are elongated capsules, which open when ripe to release the seeds, which have a pappus for anemophilous dispersal. Epilobium is native to regions of Europe and Western Asia, where it grows wild in stony soils (Gruenwald J. et al. PDR for herbal medicines, 4th Ed. Thomson, 2007).

The *Epilobium* phytocomplex contains three main classes of polyphenols: phenolic acids, flavonoids and ellagitannins, the latter being mainly represented by macrocyclic ellagitannins, such as oenothein B. The phytocomplex, the individual classes of polyphenols and, to a greater extent, oenothein B, are associated with the many health-promoting properties of this plant, including anti-inflammatory and anti-oxidant activity especially in the urological (e.g. prostatitis, benign prostatic hypertrophy, urinary tract inflammation), gastric and intestinal areas (Granica S. et al., Phytochemistry, pharmacology and traditional uses of different Epilobium species (Onagraceae): A review. Journal of Ethnopharmacology, 156, 316-346, 2014); Schepetkin IA et al. Therapeutic potential of polyphenols from Epilobium angustifolium (Fireweed). Phytother Res. 30(8):1287-97, 2016). Oenothein B can be obtained from several botanical species and not necessarily from *E*. *angustifolium L.* (US 2018/0256619).

*E. angustifolium* L. is not included in the monographs of Eur. Ph. monographs; therefore, galenic preparations of this extract refer to infusions (traditional preparations of folk medicine) and to what is available in literature and on the market. Most of the extracts are aqueous (Lesuisse D. et al., Determination of oenothein B as the active 5-alpha-reductase-inhibiting principle of the folk medicine Epilobium parviflorum; Nat Prod. May;59(5):490-2, 1996; Assessment report on *Epilobium angustifolium* L. and/or *Epilobium parviflorum* Schreb, herba, EMA, 10 March 2015; FR 2712594; DE 3605250), however the use of other solvents such as methanol, ethyl acetate, butanol and ethanol is also documented (Deng L. et al. Evaluation of the therapeutic effect against benign prostatic hyperplasia and the active constituents from Epilobium angustifolium L. Journal of Ethnopharmacology. 232:1-10, 2019; Granica S. et al., Phytochemistry, pharmacology and traditional uses of different Epilobium species (Onagraceae): A review. Journal of Ethnopharmacology, 156, 316-346, 2014). The concentration of oenothein B obtained in aqueous extracts of *Epilobium* spp. is always less than 10%.

US 6528490 discloses the anti-inflammatory activity of *E. angustifolium* extracts with an oenothein B content not exceeding 9.6% by weight. US 2018/0256619 reports that a preferred range of effective oenothein B amounts varies from 2.5 to 14% but the experimental data reported therein still refer to the extract with 9.6% oenothein B content described US 6528490.

FR 2712594 and the corresponding patent US 552594 disclose a process for the extraction of oenothein B from *Epilobium parviflorum* Schreb. with mixtures of water and water-soluble solvents, in particular acetone, and purification by reversed-phase HPLC.

WO2017108907 discloses the association between various *Epilobium* species and other plants.

The importance of oenothein B is proved by the above references. However:
1) in accordance with European regulations, purified oenothein B cannot currently be added to food supplements in Italy (Ministerial Circular of 16/06/2016) and in Europe (i.e. Novel food catalogue), while the use of *E. angustifolium* extracts, titrated and standardized in this active principle, is permitted. The extraction efficiency of this molecule in known processes is associated with a purification step.
2) the molecules present in the aqueous extracts are not representative of the phytocomplex, as the extraction yield of flavonoids is low under these conditions.

Most extracts on the market lack standardization, and known extraction and purification procedures do not provide an optimal solution to the need for extracts containing all of the most important bioactive ingredients of Epilobium, together with a high oenothein B content. Oenothein B is an ellagitannin with an antioxidant and anti-inflammatory activity. A large number of scientific studies have shown that oenothein B is able to restore the correct functionality of the prostate. In particular, a recent clinical study (Esposito C. et al., "Epilobium angustifolium L. extract with high content in oenothein B on benign prostatic hyperplasia: A monocentric, randomized, double-blind, placebo-controlled clinical trial" Biomedicine & Pharmacotherapy 138 (2021) 111414) has shown that an *E. angustifolium* extract having an oenothein B content higher than 15% by weight is significantly effective in the treatment of benign prostatic hyperplasia without renal or hepatic toxic effects.

Therefore, there is a need for process allowing the preparation of extracts that enhancing the rich phytocomplex and having a high oenothein B content to fulfil its biological and physiological function in food supplements and nutraceutical preparations.

### Description of the invention

A process for the extraction of *Epilobium* species has now been found that provides extracts with oenothein B content higher than 15% w/w, e.g. 15 to 35%, without the need for purification steps.

The process of the invention can be successfully applied to various species of *Epilobium,* in particular *E. angustifolium* and *E. parviflorum.* The use of plant material (drug) in the flowering phase ("*herba cum floribus*") is particularly preferred.

The dry extract obtainable by the process of the invention with oenothein B content ≥ 15.0% has a very rich phytocomplex, consisting of different classes of active ingredients; in particular, between 10 and 20% of phenolic acids (50% of which is represented by hydroxycinnamic acids), between 20 and 30% of flavonoids (mainly represented by flavonols) and between 40 and 60% of hydrolysable tannins, a class of compounds to which oenothein B belongs, are present.

The process of the invention for the preparation of an extract of *Epilobium* spp. with an oenothein B content higher than 15% w/w is characterized by:
- Extraction of the drug at least twice with a water:ethanol mixture in ratios ranging from 3: 1 to 1:1;
- Filtration of the percolates, evaporation of the solvent and drying of the resulting concentrated extract without further purification steps.

The drug extraction is repeated preferably 3-6 times, more preferably 4-5 times, using fresh solvent each time.

The extractions with the water:ethanol mixture are preferably carried out at a temperature ranging from 25 to 50°C for times ranging from 1 to 3 hours, at a pH ranging from 2 to 6, preferably at a pH from 2 to 3.5.

The weight ratio of drug (aerial parts of the plant) to water: ethanol mixture preferably ranges from 1:3 to 1:10.

Drying of the extraction concentrate with water:ethanol can be carried out by any known technique, e.g. by spray-drying.

According to a preferred aspect of the invention, the process further comprises an extraction step with water at acid pH of the drug subjected to the extractions with water and ethanol, filtration of the percolate, concentration of the aqueous phase, drying and addition of the dried aqueous extract to the dried extracts obtained from the previous process.

Therefore, according to this preferred aspect, the process of the invention comprises:
- A. Extraction of the drug at least twice with a water: ethanol mixture in ratios ranging from 3: 1 to 1: 1 at acid pH; filtration of the percolates, evaporation of the solvent and drying of the resulting concentrated extract without further purification steps;
- B. Extraction with water at acid pH of the drug subjected to the extractions with water and ethanol, filtration of the percolate, concentration of the aqueous phase, drying and addition of the dried aqueous extract to the dried extracts obtained from step A above.

The two fractions obtained from steps A and B, with different metabolic profiles, are analyzed and mixed appropriately until a product with the desired concentration of oenothein B is obtained.

Drying of the concentrate from the extraction with acidic pH water can be carried out by any known technique, e.g. by evaporation under reduced pressure.

The process of the invention does not involve purification and is therefore simpler and cheaper than known methods.

Analysis of the oenothein B titre is performed by HPLC-UV (method comparable to that described by Kaškonienė V, et al. (2015). "Evaluation of phytochemical composition of fresh and dried raw material of introduced Chamerion angustifolium L. using chromatographic, spectrophotometric and chemometric techniques", Phytochemistry; 115:184-93).

The method, allowing the determination of the content of the oenothein B in the dry extract, is a chromatographic separation through HPLC-DAD, using a reference standard. The sample is solubilized in an appropriate solvent and, after filtration, it is injected in HPLC. The mobile phase is acid water and acid methanol with a gradient separation on a RP-C18 column at the wavelength of 254 nm.

The method specificity allows the determination of oenothein B without the interference of other extract substances. The method is linear in the concentration range of 0.1 mg/ml and 0.7 mg/ml and the accuracy allows to estimate the average recovery % of all concentrations, that is compliant to the acceptance criterion.

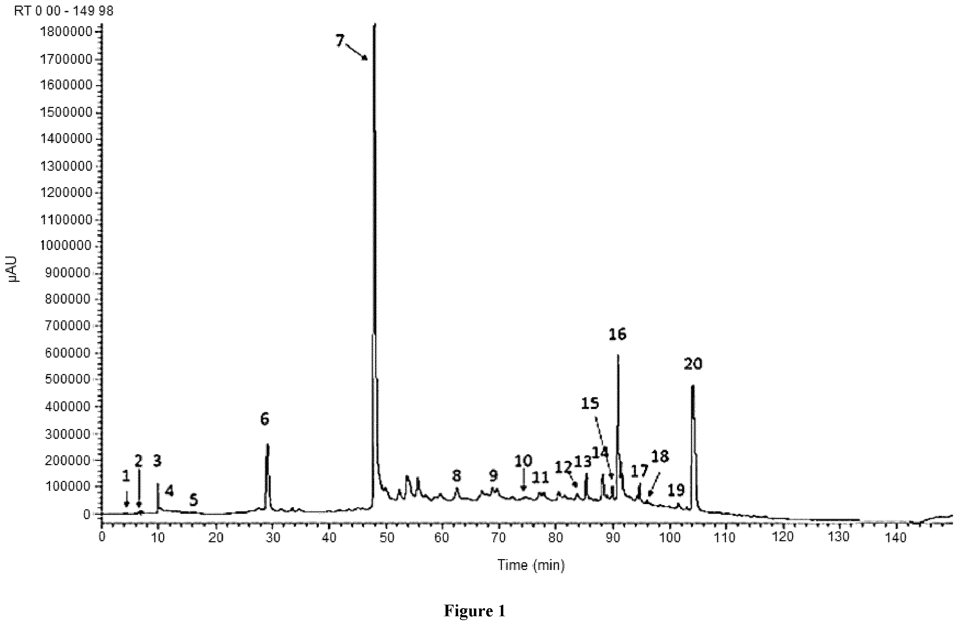

Table 1 shows the results of chemical profiling of the phytocomplex by RP-HPLC-PDA-ESI-MSn. The chromatogram is shown in Figure 1.

**Table 1.**

| **Peak** | **RT** | **Compound** | **λ Max (nm)** | ***m*/*z* [M-H]⁻** | **Fragment** |
|---|---|---|---|---|---|
| 1 | 5.18 | Hexose | 236, 272 | 225 | 179 (100), 161 (5), 143 (5) |
| 2 | 6.04 | Disaccharide | 264, 298 | 387 | 341 (100), 179 (5), 161 (10) |
| 3 | 10.25 | Gluconic acid | 212, 299 | 195 | 129 (100), 159 (20), 177 (45), 75 (10), 99 (10) |
| 4 | 12,32 | Quinic acid | 216, 292 | 191 | 127 (100), 173 (100), 85 (90), 111 (50), 93 (50) |
| 5 | 15.88 | Malic Acid | 214, 343 | 133 | 115 (100) |
| 6 | 29.43 | Gallic acid | 221, 269 | 169 | 125 (100) |
| 7 | 47.96 | Oenothein B | 213, 263 | 783 | 765 (100), 935 (20) |
| 8 | 62.53 | 1-O-Caffeylquinic acid | 210, 262, 321 | 353 | 191 (100), 179 (80), 135 (20) |
| 9 | 69.56 | 3-O-Caffeylquinic acid | 210, 262, 321 | 353 | 191 (100), 179 (15), 135 (5) |
| 10 | 74.58 | Dehydro catechin type B | 216, 271 | 439 | 393 (100) |
| 11 | 76.98 | 4-O-Caffeylquinic acid | 210, 262, 321 | 353 | 179 (95), 173 (100), 135 (20), 191 (25) |
| 12 | 83.61 | Myricetin hexoside | 204, 267, 377 | 479 | 316 (100), 287 (5), 179 (10) |
| 13 | 85.18 | Quercetin galloyl hexoside | 265, 345 | 615 | 463 (100), 301 (20) |
| 14 | 88.17 | Quercetin-hexoside | 226, 268, 285 | 463 | 301 (100) |
| 15 | 89.81 | Quercetin-3 -O-pentoside | 204, 258, 340 | 433 | 300 (100), 271 (5), 255 (5), 151 (5) |
| 16 | 90.73 | Kaempferol - hexoside | 214, 233, 278 | 447 | 285 (100) |
| 17 | 94.55 | Kaempferol -3-O-rhamnoside | 204, 264, 287 | 431 | 285 (100), 255 (5), 151 (5) |
| 18 | 95.79 | Kaempferol-p-cumaroylglucoside | 227, 266, 315 | 593 | 447 (20), 285 (100), 175 (40) |
| 19 | 101.45 | Myricetin-3-O-glucuronide | 204, 261, 357 | 493 | 317 (100) |
| 20 | 103.96 | Quercetin glucuronide | 226, 255, 349 | 477 | 301 (100) |

The invention is illustrated in more detail in the following examples.

### Example 1

### Process A: extraction with hydroalcoholic mixture

In a 2-litre reactor, 50 g of drug is introduced and 250 g of hydroalcoholic solution in a water: ethanol ratio =3:1 at acid pH is added; the extraction mixture is heated to 50°C and maintained in extraction by percolation for 2.5 hours. At the end of this first step, the obtained liquid extract is removed and the drug extracted three more times under the same conditions with the same solvent. At the end of the extraction, all the percolates are filtered, combined and the solvent is evaporated under reduced pressure at a temperature of 50°C until a concentrated extract is obtained. Final drying is carried out by a spray dryer. The amount of dry extract obtained is 12.6 g.

### Process B: extraction with water

At the end of step 1, 250 g of water at acid pH is added to the drug; the extraction mixture is heated to 50°C and maintained in extraction by percolation for 1 hour. At the end of the extraction, the percolate is separated from the drug, filtered and the solvent is evaporated under reduced pressure at a temperature of 50°C until a concentrated extract is obtained. The residual water is removed by drying under reduced pressure at 50°C until a dry extract is obtained. The amount of dry extract obtained is 2 g.

The oenothein B content thus obtained is 19.47% w/w.

### Example 2

### Process A: extraction with hydroalcoholic mixture

In a 2-litre reactor, 50 g of drug is introduced and 250 g of hydroalcoholic solution in a water: ethanol ratio =1:1 at acid pH is added; the extraction mixture is heated to 50°C and maintained in extraction by percolation for 1 hour. At the end of this first step, the liquid extract obtained is removed and the drug extracted three more times under the same conditions with the same solvent. At the end of the extraction, all the percolates are filtered, combined and the solvent is evaporated under reduced pressure at a temperature of 50°C until a concentrated extract is obtained. Final drying is carried out by a spray dryer. The amount of dry extract obtained is 13.4 g.

### Process B: extraction with water

At the end of step 1, 250 g of water at acid pH is added to the drug; the extraction mixture is heated to 50°C and maintained in extraction by percolation for 1 hour. At the end of the extraction, the percolate is separated from the drug, filtered and the solvent is evaporated under reduced pressure at a temperature of 50°C until a concentrated extract is obtained. The residual water is removed by drying under reduced pressure at 50°C until a dry extract is obtained. The amount of dry extract obtained is 2 g.

The oenothein B content thus obtained is 19,17% w/w.

### Comparative Example 3

### Extraction with water

In a 100 mL flask, 500 mg of drug is introduced and 40 ml of aqueous solvent at acid pH is added; the extraction mixture is heated to 50°C and extraction is maintained for 15 minutes. The process is repeated in duplicate, extracting for a further 15 minutes. At the end of this first step, the liquid extract obtained is filtered to remove the drug. The filtrate is evaporated under reduced pressure at a temperature of 50°C until a concentrated extract is obtained. The final drying is carried out by a spray dryer. The amount of dry extract obtained is 99 mg.

The oenothein B content thus obtained is 7.86% w/w.

### Comparative Example 4

### Extraction with hydroalcoholic mixture

In a 100 mL flask, 500 mg of drug is introduced and 40 ml of water: ethanol=1:4 at weakly acidic pH is added; the extraction mixture is heated to 50°C and extraction is maintained for 15 minutes. The process is repeated in duplicate, extracting for a further 15 minutes. At the end of this first step, the liquid extract obtained is filtered to remove the drug. The filtrate is evaporated under reduced pressure at a temperature of 50°C until a concentrated extract is obtained. The final drying is carried out by a spray dryer.

The oenothein B content thus obtained is 4.25% w/w.

## Claims

1. A process for the preparation of an extract of *Epilobium* spp. with an oenothein B content higher than 15% w/w comprising:
a) extraction of the drug 3-6 times with a water:ethanol mixture in ratios ranging from 3: 1 to 1: 1 by weight;
b) filtration of the percolates, evaporation of the solvent and drying of the resulting concentrated extract without further purification steps;
c) extracting with water at acid pH the drug previously subjected to extractions with water and ethanol, filtering the percolate, concentrating the aqueous phase, drying and adding the dried aqueous extract to the dried extracts obtained in step b).

2. Process according to claim 1 wherein *Epilobium* spp. is selected from *Epilobium angustifolium* L. and *Epilobium parviflorum* Schreb.

3. Process according to one or more of claims 1 to 2 wherein extractions with the water:ethanol mixture are carried out at a temperature ranging from 25 to 50°C for times ranging from 1 to 3 hours.

4. Process according to any one or more of claims 1 to 3 wherein extractions with the water:ethanol mixture are carried out at pH ranging from 2 to 6.

5. Process according to any one or more of claims 1 to 4 wherein the drug is *"herba cum floribus*".

6. Process according to any one or more of claims 1 to 5 wherein the drug to water:ethanol mixture weight ratio ranges from 1:3 to 1:10 by weight.

7. Process according to any one or more of claims 1 to 6 wherein drying of the extractions concentrate with water:ethanol is carried out by spray-drying.

8. Process according to any one or more of claims 1 to 7 wherein drying of the concentrate from the extraction with acidic pH water is carried out by evaporation under reduced pressure.

## Patentansprüche

1. Verfahren zur Herstellung eines Extrakts von *Epilobium* spp. mit einem Gehalt an Oenothein B von mehr als 15 Gew.-%, umfassend:
a) 3- bis 6-malige Extraktion des Arzneimittels mit einem Wasser:Ethanol-Gemisch im Gewichtsverhältnis von 3:1 bis 1:1;
b) Filtration der Perkolate, Verdampfen des Lösungsmittels und Trocknen des resultierenden konzentrierten Extrakts ohne weitere Reinigungsschritte;
c) Extraktion des zuvor mit Wasser und Ethanol extrahierten Arzneimittels mit Wasser bei saurem pH-Wert, Filtrieren des Perkolats, Konzentrieren der wässrigen Phase, Trocknen und Zugabe des getrockneten wässrigen Extrakts zu den in Schritt b) erhaltenen Trockenextrakten.

2. Verfahren nach Anspruch 1, wobei *Epilobium* spp. ausgewählt ist aus *Epilobium angustifolium* L. und *Epilobium parviflorum* Schreb.

3. Verfahren nach einem oder mehreren der Ansprüche 1 bis 2, wobei die Extraktionen mit dem Wasser:Ethanol-Gemisch bei einer Temperatur im Bereich von 25 bis 50°C für Zeiten im Bereich von 1 bis 3 Stunden durchgeführt werden.

4. Verfahren nach einem oder mehreren der Ansprüche 1 bis 3, wobei die Extraktionen mit dem Wasser:Ethanol-Gemisch bei einem pH-Wert im Bereich von 2 bis 6 durchgeführt werden.

5. Verfahren nach einem oder mehreren der Ansprüche 1 bis 4, wobei das Arzneimittel *"herba cum floribus*" ist.

6. Verfahren nach einem oder mehreren der Ansprüche 1 bis 5, wobei das Gewichtsverhältnis des Arzneimittels zum Wasser:Ethanol-Gemisch im Bereich von 1:3 bis 1:10 nach Gewicht liegt.

7. Verfahren nach einem oder mehreren der Ansprüche 1 bis 6, wobei die Trocknung des Extraktionskonzentrats mit Wasser:Ethanol durch Sprühtrocknung erfolgt.

8. Verfahren nach einem oder mehreren der Ansprüche 1 bis 7, wobei die Trocknung des Konzentrats aus der Extraktion mit Wasser mit saurem pH-Wert durch Eindampfen unter vermindertem Druck durchgeführt wird.

## Revendications

1. Procédé de préparation d'un extrait *d'Epilobium* spp. avec une teneur en oenothéine B supérieure à 15 % p/p comprenant :
a) l'extraction du médicament 3 à 6 fois avec un mélange eau:éthanol dans des proportions allant de 3:1 à 1:1 en poids ;
b) la filtration des percolats, l'évaporation du solvant et le séchage de l'extrait concentré obtenu sans autre étape de purification ;
c) l'extraction avec de l'eau à pH acide du médicament préalablement soumis aux extractions à l'eau et l'éthanol, la filtration du percolat, la concentration de la phase aqueuse, le séchage et l'ajout de l'extrait aqueux séché aux extraits séchés obtenus à l'étape b).

2. Procédé selon la revendication 1, dans lequel *Epilobium* spp. est choisi parmi *Epilobium angustifolium* L. et *Epilobium parviflorum* Schreb.

3. Procédé selon une ou plusieurs des revendications 1 à 2, dans lequel les extractions avec le mélange eau:éthanol sont effectuées à une température allant de 25 à 50 °C pendant des durées allant de 1 à 3 heures.

4. Procédé selon l'une quelconque ou plusieurs des revendications 1 à 3, dans lequel les extractions avec le mélange eau:éthanol sont réalisées à un pH allant de 2 à 6.

5. Procédé selon l'une quelconque ou plusieurs des revendications 1 à 4, dans lequel le médicament est « *herba cum floribus ».*

6. Procédé selon l'une quelconque ou plusieurs des revendications 1 à 5, dans lequel le rapport pondéral du médicament au mélange eau:éthanol va de 1:3 à 1:10 en poids.

7. Procédé selon l'une quelconque ou plusieurs des revendications 1 à 6, dans lequel le séchage du concentré des extractions avec de l'eau:éthanol est effectué par séchage par pulvérisation.

8. Procédé selon l'une quelconque ou plusieurs des revendications 1 à 7, dans lequel le séchage du concentré issu de l'extraction avec de l'eau à pH acide est effectué par évaporation sous pression réduite.
